# EUROPÄISCHE PATENTSCHRIFT

(11) **EP 1 235 546 B1**
(45) Veröffentlichungstag und Bekanntmachung des Hinweises auf die Patenterteilung: **30.03.2005**
(21) Anmeldenummer: 00985096.7
(22) Anmeldetag: 25.11.2000
(51) Int. Cl.: A61K 7/00, B01F 17/00

(54) **VERWENDUNG VON NANOSKALIGEN WACHSEN**
USE OF NANOSCALE WAXES
UTILISATION DE CIRES NANOMETRIQUES

(30) Priorität: 04.12.1999 DE 19958521
(43) Veröffentlichungstag der Anmeldung: 04.09.2002
(73) Patentinhaber: Cognis Deutschland GmbH & Co. KG, 40589 Düsseldorf (DE)
(72) Erfinder: SEIPEL, Werner, 40723 Hilden (DE); BOYXEN, Norbert, 47906 Kempen (DE); HENSEN, Hermann, 42781 Haan (DE)
(86) Internationale Anmeldenummer: PCT/EP2000/011772
(87) Internationale Veröffentlichungsnummer: WO 2001/039729

(56) Entgegenhaltungen:
- EP-A- 0 506 197
- WO-A-00/10522
- WO-A-00/54733
- DE-A- 4 336 407
- DE-A- 4 441 029
- DE-A- 19 710 149
- FR-A- 2 666 015
- FR-A- 2 735 690

## Beschreibung

Die Erfindung befindet sich auf dem Gebiet der Nanopartikel und betrifft die Verwendung von nanoskaligen Wachsen mit Teilchendurchmessem im Bereich von 10 bis 300 nm als Trübungsmittel, Avivagemittel und Rückfettungsmittel zur Herstellung von oberflächenaktiven Zubereitungen.

### Stand der Technik

Kosmetische Zubereitungen lassen sich grob in Reinigungs- und Pflegemittel für Haut und Haare einteilen. Ihnen werden vielfach einen Reihe von Wachsen zugesetzt, wie beispielsweise natürliche oder synthetische Wachse, Perlglanzwachse, Silikonwachse und dergleichen. An moderne Produkte dieser Art werden vom Verbraucher immer stärker wachsende Ansprüche gestellt, von denen die ökotoxikologische Verträglichkeit, d.h. die Kompatibilität mit der Umwelt und dem Anwender, zu einer Selbstverständlichkeit geworden ist. Es besteht weiterhin ein Bedarf an Wachsen in neuen Anbietungsformen, die im Bereich der Haaravivage (Kämmbarkeit, Glanz usw.) als auch im Sektor der Hautpflege durch verbesserte sensorische Eigenschaften und rückfettende Effekte eingesetzt werden können. Weiterhin sollte die Stabilität von kosmetischen Zubereitungen dieser Art sowie eine verbesserte Konsistenz gegeben sein.

Die Aufgabe der vorliegenden Erfindung hat folglich darin bestanden, Mittel zur Verfügung zu stellen, die das oben beschriebene komplexe Anforderungsprofil erfüllen.

### Beschreibung der Erfindung

Gegenstand der Erfindung ist die Verwendung von nanoskaligen Wachsen mit Teilchendurchmessem im Bereich von 10 bis 300 nm, welche von einem Schutzkolloid ummantelt vorliegen, das ausgewählt ist aus der Gruppe Gelatine, Casein, Gummi arabicum, Lysalbinsäure, Stärke, Polyvinylalkohole, Polyvinylpyrrolidone, Polyalkylenglycole und Polyacrylate, zur Herstellung von oberflächenaktiven Zubereitungen.

Überraschenderweise wurde gefunden, daß sowohl die Stabilität von Zubereitungen, wie beispielsweise Lotionen und Cremes, als auch deren Konsistenz durch den Zusatz von Wachsen signifikant verbessert wird, wenn diese in Form von mit speziellen Schutzkolloiden ummantelten Nanoteilchen, d.h. Partikeln mit einem mittleren Durchmesser im Bereich von 10 bis 300 und vorzugsweise 50 bis 150 nm vorliegen. Gleichzeitig zeigen derartige Zubereitungen eine ausgezeichnete rückfettende Wirkung, verbesserte dermatologische Verträglichkeiten und sensorische Eigenschaften an Haut und Haar sowie eine Steigerung des Haarglanzes und - volumens. Zusätzlich bewirken diese Verbindungen auf Keratinfaser einen deutlichen avivierenden Effekt, d.h. sie verbessern die Kämmbarkeit und reduzieren die statische Aufladung zwischen den Fasern. Darüber hinaus lassen sich mit derartigen Nano-Wachsen klare, transparente und trübe Formulierungen herstellen.

### Wachse

Unter Wachsen sind **natürliche** oder **synthetische** Stoffe zu verstehen, welche bei 20°C knetbar, fest bis brüchig hart, grob bis feinkristallin, durchscheinend bis opak, jedoch nicht glasartig sind, oberhalb von 40°C ohne sich zu zersetzen schmelzen, schon wenig oberhalb des Schmelzpunktes niedrigviskos und nicht fadenziehend sind. Die im Sinne der Erfindung einzusetzenden Wachse unterscheiden sich beispielsweise von Harzen dadurch, daß sie in der Regel etwa zwischen und 50 und 90°C, in Ausnahmefällen auch bis zu 200°C, in den schmelzflüssigen, niedrigviskosen Zustand übergehen und praktisch frei von aschebildenden Verbindungen sind. Nach ihrer Herkunft teilt man die Wachse in die folgenden drei Gruppen ein: Natürlich Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse. In diesem Zusammenhang ist der Einsatz von natürlichen Wachsen wie beispielsweise Bienenwachs, speziell von pflanzlichen Wachsen bevorzugt.

Als Wachse können weiterhin **Wachsester** eingesetzt werden , die der Formel **(I)** folgen,

**R**^{**1**}**COO-R**^{**2**} (I)

in der R¹CO für einen linearen oder verzweigten Acylrest mit 6 bis 22 Kohlenstoffatomen mit 0 und/oder 1 bis 3 Doppelbindungen und R² für einen linearen oder verzweigten Alkyl- und/oder Alkenylrest mit 6 bis 22 Kohlenstoffatomen steht, mit der Maßgabe, daß die Anzahl der Kohlenstoffatome im Ester mindestens 20 beträgt. Typische Beispiele hierfür sind Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Vorzugsweise werden ungesättigte Wachsester, wie beispielsweise Cetylpalmitat, Stearylstearat, Oleyloleat und Oleylerucat eingesetzt.

Als Wachse kommen ebenfalls **Perlglanzwachse** in Frage, wie beispielsweise Alkylenglycolester; Fettsäurealkanolamide; Partialglyceride; Ester von mehrwertigen, gegebenenfalls hydroxysubstituierte Carbonsäuren mit Fettalkoholen mit 6 bis 22 Kohlenstoffatomen; Fettstoffe, wie beispielsweise Fettalkohole, Fettketone, Fettaldehyde, Fettether und Fettcarbonate, die in Summe mindestens 24 Kohlenstoffatome aufweisen; Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.
- **Alkylenglycolester.** Bei den Alkylenglycolestem handelt es sich üblicherweise um Mono- und/oder Diester von Alkylenglycolen, die der Formel **(II)** folgen,

   **R**^{**3**}**CO(OA)**_{**n**}**OR**^{**4**} (II)

   in der R³CO für einen linearen oder verzweigten, gesättigten oder ungesättigten Acylrest mit 6 bis 22 Kohlenstoffatomen, R⁴ für Wasserstoff oder R³CO und A für einen linearen oder verzweigten Alkylenrest mit 2 bis 4 Kohlenstoffatomen und n für Zahlen von 1 bis 5 steht. Typische Beispiele sind Mono- und/oder Diester von Ethylenglycol, Propylenglycol, Diethylenglycol, Dipropylenglycol, Triethylenglycol oder Tetraethylenglycol mit Fettsäuren mit 6 bis 22, vorzugsweise 12 bis 18 Kohlenstoffatomen als da sind: Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt ist der Einsatz von Ethylenglycolmono- und/oder -distearat.
- **Fettsäurealkanolamide.** Fettsäurealkanolamide, die als Perlglanzwachse in Frage kommen, folgen der Formel **(III),**

   **R**^{**6**}**CO-NR**^{**5**}**-B-OH** (III)

   in der R⁶CO für einen linearen oder verzweigten, gesättigten oder ungesättigten Acylrest mit 6 bis 22 Kohlenstoffatomen, R⁵ für Wasserstoff oder einen gegebenenfalls hydroxysubstituierten Alkylrest mit 1 bis 4 Kohlenstoffatomen und B für eine lineare oder verzweigte Alkylengruppe mit 1 bis 4 Kohlenstoffatomen steht. Typische Beispiele sind Kondensationsprodukte von Ethanolamin, Methylethanolamin, Diethanolamin, Propanolamin, Methylpropanolamin und Dipropanolamin sowie deren Mischungen mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt ist der Einsatz von Stearinsäureethanolamid.
- **Partialglyceride.** Partialglyceride, die über Perlglanzeigenschaften verfügen, stellen Mono- und/oder Diester des Glycerins mit Fettsäuren, nämlich beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen dar. Sie folgen der Formel **(IV)**, 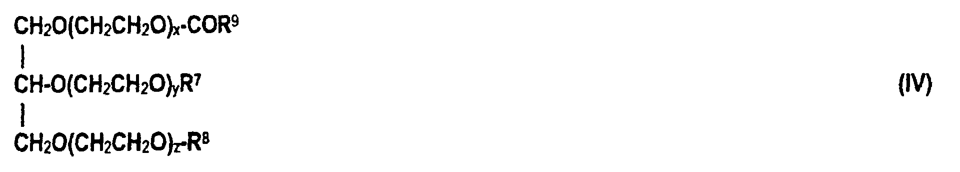 in der R⁹CO für einen linearen oder verzweigten Acylrest mit 6 bis 22 Kohlenstoffatomen, R⁷ und R⁸ unabhängig voneinander für Wasserstoff oder R⁹CO, x, y und z in Summe für 0 oder für Zahlen von 1 bis 30 und X für ein Alkali- oder Erdalkalimetall mit der Maßgabe steht, daß mindestens einer der beiden Reste R⁷ und R⁸ Wasserstoff darstellt. Typische Beispiele sind Laurinsäuremonoglycerid, Laurinsäurediglycerid, Kokosfettsäuremonoglycerid, Kokosfettsäuretriglycerid, Palmitinsäuremonoglycerid, Palmitinsäuretriglycerid, Stearinsäuremonoglycerid, Stearinsäurediglycerid, Isostearin-säuremonoglycerid, Isostearinsäurediglycerid, Ölsäuremonoglycerid, Ölsäurediglycerid, Talgfettsäuremonoglycerid, Talgfettsäurediglycerid, Behensäuremonoglycerid, Behensäurediglycerid, Erucasäure-monoglycerid, Erucasäurediglycerid sowie deren technische Gemische, die untergeordnet aus dem Herstellungsprozeß noch geringe Mengen an Triglycerid enthalten können.
- **Mehrwertige Carbonsäure- und Hydroxycarbonsäureester.** Als Perlglanzwachse kommen weiterhin Ester von mehrwertigen, gegebenenfalls hydroxysubstituierten Carbonsäuren mit Fettalkoholen mit 6 bis 22 Kohlenstoffatomen in Frage. Als Säurekomponente dieser Ester kommen beispielsweise Malonsäure, Maleinsäure, Fumarsäure, Adipinsäure, Sebacinsäure, Azelainsäure, Dodecandisäure, Phthalsäure, Isophthalsäure und insbesondere Bemsteinsäure sowie Äpfelsäure, Citronensäure und insbesondere Weinsäure und deren Mischungen in Betracht. Die Fettalkohole enthalten 6 bis 22, vorzugsweise 12 bis 18 und insbesondere 16 bis 18 Kohlenstoffatome in der Alkylkette. Typische Beispiele sind Capronalkohol, Caprylalkohol, 2-Ethylhexylalkohol, Caprinalko-hol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol. Erucylalkohol und Brassidylalkohol sowie deren technische Mischungen. Die Ester können als Voll- oder Partialester vorliegen, vorzugsweise werden Mono- und vor allem Diester der Carbon- bzw. Hydroxycarbonsäuren eingesetzt. Typische Beispiele sind Bemsteinsäuremono- und -dilaurylester, Bemsteinsäuremono- und - dicetearlyester, Bemsteinsäuremono- und -distearylester, Weinsäuremono- und -dilaurylester, Weinsäuremono- und dikokosalkylester, Weinsäuremono- und -dicetearylester, Citronensäuremono-, -di- und -trilaurylester, Citronensäuremono-, -di- und -trikokosalkylester sowie Citronensäuremono-, -di- und -tricetearylester.
- **Fettalkohole.** Als weitere Gruppe von Perlglanzwachsen können langkettige Fettalkohole eingesetzt werden, die der Formel **(V)** folgen,

   **R**^{**10**}**OH** (V)

   in der R¹⁰ für einen linearen Alkylrest mit 24 bis 48, vorzugsweise 32 bis 36 Kohlenstoffatomen steht. Bei den genannten Stoffen handelt es sich in der Regel um Oxidationsprodukte langkettiger Paraffine.
- **Fettketone.** Fettketone, die als Komponente (a) in Betracht kommen, folgen vorzugsweise der Formel **(VI)**,

   **R**^{**12**}**-CO-R**^{**11**} (VI)

   in der R¹² und R¹¹ unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Die Ketone können nach Verfahren des Stands der Technik hergestellt werden, beispielsweise durch Pyrolyse der entsprechenden Fettsäure-Magnesiumsalze. Die Ketone können symmetrisch oder unsymmetrisch aufgebaut sein, vorzugsweise unterscheiden sich die beiden Reste R¹² und R¹¹ aber nur um ein Kohlenstoffatom und leiten sich von Fettsäuren mit 16 bis 22 Kohlenstoffatomen ab. Dabei zeichnet sich Stearon durch besonders vorteilhafte Perlglanzeigenschaften aus.
- **Fettaldehyde.** Als Perlglanzwachse geeignete Fettaldehyde entsprechen der Formel **(VII),**

   **R**^{**13**}**COH** (VII)

   in der R¹³CO für einen linearen oder verzweigten Acylrest mit 24 bis 48, vorzugsweise 28 bis 32 Kohlenstoffatomen steht.
- **Fettether.** Als Perlglanzwachse kommen ferner Fettether der Formel **(VIII)** in Frage,

   **R**^{**15**}**-O-R**^{**14**} (VIII)

   in der R¹⁵ und R¹⁴ unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Fettether der genannten Art werden üblicherweise durch saure Kondensation der entsprechenden Fettalkohole hergestellt. Fettether mit besonders vorteilhaften Perlglanzeigenschaften werden durch Kondensation von Fettalkoholen mit 16 bis 22 Kohlenstoffatomen, wie beispielsweise Cetylalkohol, Cetearylalkohol, Stearylalkohol, Isostearylalkohol, Oleylalkohol, Behenylalkohol und/oder Erucylalkohol erhalten.
- **Fettcarbonate.** Als Perlglanzwachse kommen weiterhin Fettcarbonate der Formel **(IX)** in Betracht,

   **R**^{**17**}**O-CO-OR**^{**16**} (IX)

   in der R¹⁷ und R¹⁶ unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Die Stoffe werden erhalten, indem man beispielsweise Dimethyl- oder Diethylcarbonat mit den entsprechenden Fettalkoholen in an sich bekannter Weise umestert. Demzufolge können die Fettcarbonate symmetrisch oder unsymmetrisch aufgebaut sein. Vorzugsweise werden jedoch Carbonate eingesetzt, in denen R¹⁵ und R¹⁶ gleich sind und für Alkylreste mit 16 bis 22 Kohlenstoffatomen stehen. Besonders bevorzugt sind Umesterungsprodukte von Dimethyl- bzw. Diethylcarbonat mit Cetylalkohol, Cetearylalkohol, Stearylalkohol, Isostearylalkohol, Oleylalkohol, Behenylalkohol und/ oder Erucylalkohol in Form ihrer Mono- und Diester bzw. deren technischen Mischungen.
- **Epoxidringöffnungsprodukte.** Bei den Ringöffnungsprodukten handelt es sich um bekannte Stoffe, die üblicherweise durch säurekatalysierte Umsetzung von endständigen oder innenständigen Olefinepoxiden mit aliphatischen Alkoholen hergestellt werden. Die Reaktionsprodukte folgen vorzugsweise der Formel **(X),** 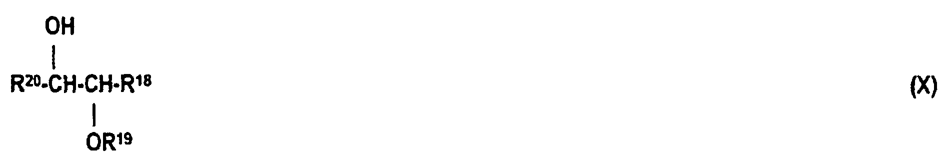 in der R²⁰ und R¹⁸ für Wasserstoff oder einen Alkylrest mit 10 bis 20 Kohlenstoffatomen steht, mit der Maßgabe, daß die Summe der Kohlenstoffatome von R²⁰ und R¹⁸ im Bereich von 10 bis 20 liegt und R¹⁹ für einen Alkyl- und/oder Alkenylrest mit 12 bis 22 Kohlenstoffatomen und/oder den Rest eines Polyols mit 2 bis 15 Kohlenstoffatomen und 2 bis 10 hydroxylgruppen steht. Typische Beispiele sind Ringöffnungsprodukte von α-Dodecenepoxid, α-Hexadecenepoxid, α-Octadecenepoxid, α-Eicosenepoxid, α-Docosenepoxid, i-Dodecenepoxid, i-Hexadecenepoxid, i-Octadecenepoxid, i-Eicosenepoxid und/oder i-Docosenepoxid mit Laurylalkohol, Kokosfettalkohol, Myristyl-alkohol, Cetylalkohol, Cetearylalkohol, Stearylalkohol, Isostearylalkohol, Oleylalkohol, Elaidylalkohol, Petroselinylalkohol, Linolylalkohol, Linolenylalkohol, Behenylalkohol und/oder Erucylalkohol. Vorzugsweise werden Ringöffnungsprodukte von Hexa- und/oder Octadecenepoxiden mit Fettalko-holen mit 16 bis 18 Kohlenstoffatomen eingesetzt. Werden anstelle der Fettalkohole Polyole für die Ringöffnung eingesetzt, so handelt es sich beispielsweise um folgende Stoffe: Glycerin; Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methyol-verbindungen, wie insbesondere Trimethylolethan, Trimethylolpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin.

Die Einsatzmenge der Perlglanzwachse kann - bezogen auf die Mittel - bei 0,1 bis 5, vorzugsweise 0,5 bis 3 und insbesondere 1 bis 1,5 Gew.-% liegen.

### Siliconwachse

Geeignete Wachse sind weiterhin Siliconverbindungen wie beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur harzförmig vorliegen.

### Herstellung von Nanopartikeln

Ein Verfahren zur Herstellung von Nanoteilchen durch rasche Entspannung von überkritischen Lösungen **(Rapid Expansion of Supercritical Solutions RESS)** ist beispielsweise aus dem Aufsatz von S.Chihlar, M.Türk und K.Schaber in **Proceedings World Congress on Particle Technology 3,** **Brighton, 1998** bekannt. In einer bevorzugten Ausführungsform der Erfindung setzt man nanoskalige Wachse ein, die man erhält, indem man
(a) die Ausgangsstoffe unter überkritischen oder nahekritischen Bedingungen in einem geeigneten Lösungsmittel löst,
(b) die fluide Mischung über eine Düse in ein Vakuum, ein Gas oder eine Flüssigkeit entspannt, und
(c) das Lösemittel dabei gleichzeitig verdampft.

Um zu verhindern, daß die Nanoteilchen wieder zusammenbacken, ist es notwendig, die Ausgangsstoffe in Gegenwart bestimmter Schutzkolloide zu lösen und/oder die kritischen Lösungen in wäßrige und/oder alkoholische Lösungen der Schutzkolloide oder aber in kosmetische Öle zu entspannen, welche ihrerseits wieder gelöste Schutzkolloide enthalten können. Erfindungsgemäß geeignete Schutzkolloide sind Gelatine, Casein, Gummi arabicum, Lysalbinsäure, Stärke, Polyvinylalkohole, Polyvinylpyrrolidone, Polyalkylenglycole und Polyacrylate. Üblicherweise werden die Schutzkolloide in Mengen von 0,1 bis 20, vorzugsweise 5 bis 15 Gew.-% - bezogen auf die Wachse - eingesetzt.

Ein weiteres geeignetes Verfahren zur Herstellung der nanoskaligen Teilchen bietet die **Evaporationstechnik.** Hierbei werden die Ausgangsstoffe zunächst in einem geeigneten organischen Lösungsmittel (z.B. Alkane, pflanzliche Öle, Ether, Ester, Ketone, Acetale und dergleichen) gelöst. Anschließend werden die Lösungen derart in Wasser oder einem anderen Nicht-Lösungsmittel, gegebenenfalls in Gegenwart einer darin gelösten oberflächenaktiven Verbindung gegeben, daß es durch die Homogenisierung der beiden nicht miteinander mischbaren Lösungsmittel zu einer Ausfällung der Nanoteilchen kommt, wobei das organische Lösungsmittel vorzugsweise verdampft. Anstelle einer wäßrigen Lösung können auch O/W-Emulsionen bzw. O/W-Mikroemulsionen eingesetzt werden. Als oberflächenaktive Verbindungen können die bereits eingangs erläuterten Emulgatoren und Schutzkolloide verwendet werden. Eine weitere Möglichkeit zur Herstellung von Nanoteilchen besteht in dem sogenannten **GAS-Verfahren** (Gas Anti Solvent Recrystallization). Das Verfahren nutzt ein hochkomprimiertes Gas oder überkritisches Fluid (z.B. Kohlendioxid) als Nicht-Lösungsmittel zur Kristallisation von gelösten Stoffen. Die verdichtete Gasphase wird in die Primärlösung der Ausgangsstoffe eingeleitet und dort absorbiert, wodurch sich das Flüssigkeitsvolumen vergrößert, die Löslichkeit abnimmt und feinteilige Partikel ausgeschieden werden. Ähnlich geeignet ist das **PCA-Verfahren** (Precipitation with a Compressed Fluid Anti-Solvent). Hier wird die Primärlösung der Ausgangsstoffe in ein überkritisches Fluid eingeleitet, wobei sich feinstverteilte Tröpfchen bilden, in denen Diffusionsvorgänge ablaufen, so daß eine Ausfällung feinster Partikel erfolgt. Beim **PGSS-Verfahren** (Particles from Gas Saturated Solutions) werden die Ausgangsstoffe durch Aufpressen von Gas (z.B. Kohlendioxid oder Propan) aufgeschmolzen. Druck und Temperatur erreichen nahe- oder überkritische Bedingungen. Die Gasphase löst sich im Feststoff und bewirkt eine Absenkung der Schmelztemperatur, der Viskosität und der Oberflächenspannung. Bei der Expansion durch eine Düse kommt es durch Abkühlungseffekte zur Bildung feinster Teilchen.

Ein weiteres Verfahren stellt das schnelle Abkühlen von in der Wärme gelösten bzw. feinst verteilten Wachspartikeln dar. Das schnelle Durchschreiten des Kristallisationstemperaturbereiches der verschiedenen Wachse ist der prozeßbestimmende Schritt.

### Gewerbliche Anwendbarkeit

Gegenüber Wachsen des Stands der Technik bewirkt die besondere Feinteiligkeit der Partikel eine erhöhte Stabilität und Konsistenz von Zubereitungen sowie einen avivierenden Effekt an Keratinfasem. (Antisplisswirkung) Weiterhin zeigen sie einen rückfettenden Effekt, trübende Eigenschaften, sowie eine bessere Fixierbarkeit dieser Wachse an Haut und Haar. Außerdem ist die dermatologische Verträglichkeit dieser Formulierungen durch eine Anschirmung der Haut erhöht. Weitere Gegenstände der vorliegenden Erfindung betreffen daher die Verwendung der nanoskaligen Wachse als Avivagemittel zur Herstellung in Haarbehandlungsmitteln als Trübungsmittel, und/oder als Rückfettungsmittel zur Herstellung kosmetischen und/oder pharmazeutischen Zubereitungen, insbesondere in Haar- und/oder Hautbehandlungsmitteln. Die Einsatzmenge der Wachse liegt dabei üblicherweise in der Größenordnung von 0,1 bis 5, vorzugsweise 0,5 bis 3 und insbesondere 1 bis 2 Gew.-% - bezogen auf die Zubereitungen -.

### Oberflächenaktive Zubereitungen

Die erfindungsgemäß zu verwendenden nanoskaligen Wachse können zur Herstellung von oberflächenaktiven Zubereitungen, wie Spül- und Reinigungsmittel sowie kosmetische und/oder pharmazeutische Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Überfettungsmittel, Konsistenzgeber, Verdickungsmittel, Polymere, Siliconverbindungen, Fette, Lecithine, Phospholipide, Stabilisatoren, biogene Wirkstoffe, Deodorantien, Antitranspirantien, Antischuppenmittel, Filmbildner, Quellmittel, UV-Lichtschutzfaktoren, Antioxidantien, Hydrotrope, Konservierungsmittel, Insektenrepellentien, Selbstbräuner, Tyrosininhibitoren (Depigmentierungsmittel), Solubilisatoren, Parfümöle, Farbstoffe und dergleichen enthalten.

Typische Beispiele für geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle und/oder aliphatische bzw. naphthenische Kohlenwasserstoffe, wie z.B. wie Squalan, Squalen oder Dialkylcyclohexane in Betracht.

Als Emulgatoren kommen beispielsweise nichtionogene Tenside aus mindestens einer der folgenden Gruppen in Frage:
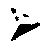 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
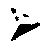 Alkyl- und/oder Alkenyloligoglykoside mit 8 bis 22 Kohlenstoffatomen im Alk(en)ylrest und deren ethoxylierte Analoga;
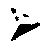 Anlagerungsprodukte von 1 bis 15 Mol Ethylenoxid an Ricinusöl und/oder gehärtetes Ricinusöl;
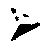 Anlagerungsprodukte von 15 bis 60 Mol Ethylenoxid an Ricinusöl und/oder gehärtetes Ricinusöl;
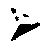 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
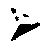 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
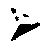 Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin.
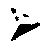 Mono-, Di- und Trialkylphosphate sowie Mono-, Di- und/oder Tri-PEG-alkylphosphate und deren Salze;
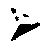 Wollwachsalkohole;
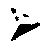 Polysiloxan-Polyalkyl-Polyether-Copolymere bzw. entsprechende Derivate;
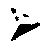 Polyalkylenglycole sowie
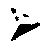 Glycerincarbonat.

Die **Anlagerungsprodukte von Ethylenoxid und/oder von Propylenoxid** an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

**Alkyl- und/oder Alkenyloligoglycoside,** ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid. Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als **Sorbitanester** kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete **Polyglycerinester** sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Beispiele für weitere geeignete **Polyolester** sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren **zwitterionische Tenside** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Schließlich kommen auch **Kationtenside** als Emulgatoren in Betracht, wobei solche vom Typ der Esterquats, vorzugsweise methylquatemierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron). Polyaminopolyamide, wie z.B. beschrieben in der **FR 2252840 A** sowie deren vemetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropyl-methacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride. Neben den Fetten kommen als Zusatzstoffe auch fettähnliche Substanzen, wie **Lecithine** und **Phospholipide** in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet und folgen der allgemeinen Formel 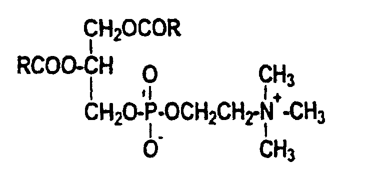 wobei R typischerweise für lineare aliphatische Kohlenwasserstoffreste mit 15 bis 17 Kohlenstoffatomen und bis zu 4 cis-Doppelbindungen steht. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren** sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Apfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen.

Wässrige oder wasserfreie Formulierungen von Antitranspirantien enthalten typischerweise folgende Inhaltsstoffe:
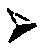 adstringierende Wirkstoffe,
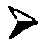 Ölkomponenten,
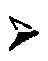 nichtionische Emulgatoren,
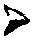 Coemulgatoren,
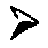 Konsistenzgeber,
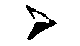 Hilfsstoffe wie z. B. Verdicker oder Komplexierungsmittel und/oder
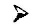 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin.
Daneben können in Antitranspirantien übliche öllösliche und wasserlösliche Hilfsmittel in geringeren Mengen enthalten sein. Solche öllöslichen Hilfsmittel können z.B. sein:
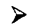 entzündungshemmende, hautschützende oder wohlriechende ätherische Öle,
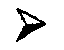 synthetische hautschützende Wirkstoffe und/oder
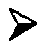 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als **Antischuppenmittel** können Octopirox® (1-Hydroxy-4-methyl-6-(2,4,4-trimythylpentyl)-2-(1H)-pyridon-monoethanolaminsalz), Baypival, Pirocton Olamin, Ketoconazol®, (4-Acetyl-1-{-4-[2-(2.4-dichlorphenyl)r-2-(1H-imidazol-1-ylmethyl)-1,3-dioxylan-c-4-ylmethoxyphenyl}piperazin, Selendisulfid, Schwefel kolloidal, Schwefelpolyehtylenglykolsorbitanmonooleat, Schwefelrizinolpolyehtoxylat, Schwefelteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat, Zinkpyrethion, Aluminiumpyrition und Magnesiumpyrithion / Dipyrithion-Magnesiomsulfat eingesetzt werden.

Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
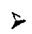 3-Benzylidencampher bzw. 3-Benzylidennorcampher und dessen Derivate, z.B. 3-(4-Methylbenzyliden)campher wie in der **EP 0693471 B1** beschrieben;
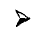 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
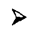 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
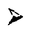 Ester der Salicylsäure, vorzugsweise Salicylsäure-2-ethylhexylester, Salicylsäure-4-isopropylbenzylester, Salicylsäurehomomenthylester;
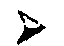 Derivate des Benzophenons, vorzugsweise 2-Hydroxy-4-methoxybenzophenon, 2-Hydroxy-4-methoxy-4'-methylbenzophenon, 2,2'-Dihydroxy4-methoxybenzophenon;
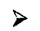 Ester der Benzalmalonsäure, vorzugsweise 4-Methoxybenzmalonsäuredi-2-ethylhexylester;
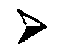 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
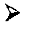 Propan-1,3-dione, wie z.B. 1-(4-tert.Butylphenyl)-3-(4'methoxyphenyl)propan-1,3-dion;
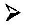 Ketotricyclo(5.2.1.0)decan-Derivate, wie in der **EP 0694521 B1** beschrieben.

Als wasserlösliche Substanzen kommen in Frage:
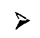 2-Phenylbenzimidazol-5-sulfonsäure und deren Alkali-, Erdalkali-, Ammonium-, Alkylammonium-, Alkanolammonium- und Glucammoniumsalze;
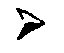 Sulfonsäurederivate von Benzophenonen, vorzugsweise 2-Hydroxy-4-methoxybenzophenon-5-sulfonsäure und ihre Salze;
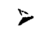 Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinoi und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
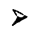 Glycerin;
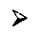 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
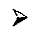 technische Oligoglyceringemische mit einem Eigenkondensationsgrad von 1,5 bis 10 wie etwa technische Diglyceringemische mit einem Diglyceringehalt von 40 bis 50 Gew.-%;
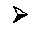 Methyolverbindungen, wie insbesondere Trimethylolethan, Trimethylolpropan, Trimethylolbutan, Pentaerythrit und Dipentaerythrit;
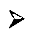 Niedrigalkylglucoside, insbesondere solche mit 1 bis 8 Kohlenstoffen im Alkylrest, wie beispielsweise Methyl- und Butylglucosid;
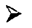 Zuckeralkohole mit 5 bis 12 Kohlenstoffatomen, wie beispielsweise Sorbit oder Mannit,
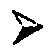 Zucker mit 5 bis 12 Kohlenstoffatomen, wie beispielsweise Glucose oder Saccharose;
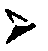 Aminozucker, wie beispielsweise Glucamin;
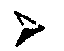 Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Ethyl Butylacetylaminopropionate in Frage, als **Selbstbräuner** eignet sich Dihydroxyaceton. Als **Tyrosinhinbitoren,** die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzem (Pinien-, Sandel-, Guajak-, Zedern-, Rosenholz), Kräutern und Gräsem (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romüllat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

Zur Herstellung der nanoskaligen Wachsen (Beispiele 1 bis 4 und 6 - 8) wurde zunächst Kohlendioxid einem Reservoir mit einem konstanten Druck von 60 bar entnommen und über eine Kolonne mit einer Aktivkohle- und einer Molekularsieb-Packung gereinigt. Nach der Verflüssigung wurde das CO₂ mit Hilfe einer Diaphragma-Pumpe bei einer konstanten Fördermenge von 3,5 l/h auf den gewünschten überkritischen Druck p verdichtet. Anschließend wurde das Lösungsmittel in einem Vorheizer auf die erforderliche Temperatur T1 gebracht und in eine Extraktionskolonne (Stahl, 400 ml) geleitet, welche mit den Wachsen beladen war. Die resultierende überkritische, d.h. fluide Mischung wurde über eine lasergezogene Düse (Länge 830 µm, Durchmesser 45 µm) bei einer Temperatur T2 in eine Plexiglas Expansionskammer versprüht, die eine 4 Gew.-%ige wäßrige Lösung eines Schutzkolloids enthielt. Das fluide Medium verdampfte und zurück blieben die im Schutzkolloid eingeschlossenen, dispergierten Nanopartikel. Die Verfahrensbedingungen und der mittlere Partikelgrößenbereich (photometrisch nach der 3-WEM-Methode bestimmt) sind in der nachfolgenden Tabelle 1 angegeben.

**Tabelle 1:**

| **Nanopartikel** | | | | | | | |
|---|---|---|---|---|---|---|---|
| **Bsp.** | **Wachse** | **Lsgm.** | **p** **bar** | **T1** **°C** | **T2** **°C** | **Schutzkolloid** | **PGB** **nm** |
| 1 | **Cutina ® AGS** Ethylenglycolbisstearat | CO₂ | 200 | 80 | 175 | Polyvinylalkohol | 60-120 |
| 2 | **Cutina® STE** Distearylether | CO₂ | 180 | 70 | 160 | Polyethylenglycol (M=400) | 75-120 |
| 3 | **Stearon** | CO₂ | 200 | 85 | 180 | Polyvinylalkohol | 75-130 |
| 4 | **Bienenwachs** | CO₂ | 200 | 85 | 175 | Polyvinylalkohol | 60-140 |
| 6 | **Sonnenblumenwachs** | CO₂ | 200 | 85 | 175 | Polyvinylalkohol | 60-140 |
| 7 | **Lanolin** | CO₂ | 200 | 85 | 175 | Polyvinylalkohol | 60-140 |
| 8 | **Generol® 122 N** Raffiniertes Sojastearin | CO₂ | 200 | 85 | 175 | Polyvinylalkohol | 60-140 |

Zur Herstellung eines Wachscompounds werden Wachspartikel bei 60 - 80 °C in den in Tabelle 2 aufgeführten Komponenten gelöst und schnell auf 25 °C abgekühlt.

**Tabelle 2:**

| **Nanoskaliges Wachscompound - Mengenangaben in Gew.-% -** | | | | | | |
|---|---|---|---|---|---|---|
| **Zusammensetzung** | **a** | **b** | **c** | **d** | **e** | **f** |
| **Lanolin (gemäß Tabelle 1, Beispiel 7)** | 20 | 10 | 10 | 10 | 15 | 20 |
| **Texapon N70** Natrium Laureth Sulfat | 10 | 10 | 10 | 10 | - | - |
| **Dehyton® PK 45** Cocamidopropyl Betaine | 10 | - | - | - | - | 10 |
| **Plantacare® 818** Coco Glucosides | - | - | - | 10 | 15 | 10 |
| **Wasser** | ad 100 | | | | | |
| **Teilchengröße [nm]** | 50 | 50 | 150 | 70 | 100 | 150 |

**Tabelle 3:**

| **Nanoskalige Wachszubereitungen - Mengenangaben in Gew.-%** | | | | | | | | |
|---|---|---|---|---|---|---|---|---|
| **Zusammensetzung** | **I** | **II** | **III** | **IV** | **V** | **VI** | **VII** | **V*** |
| **Texapon N70** Natrium Laureth Sulfaf | 11,5 | | | | | | | |
| **Dehyton® PK 45** Cocamidopropyl Betaine | 3,0 | | | | | | | |
| **Plantacare® 818** Coco Glucosides | 3,0 | | | | | | | |
| **Wachscompounds** *(Gew.-%*/*Compounds gem. Tabelle 2, Spatlen a-f)* | 5/*a* | 10/a | 10/a | 10/d | 10/d | 5/*d* | 10/*f* | - |
| **Sodium Chloride** | 3 | 2,5 | 3 | 2,8 | 2,7 | 2,5 | 2 | 3 |
| **Konservierungsmittel** | 0,1 | | | | | | | |
| **Wasser** | ad 100 | | | | | | | |
| **pH-Wert** | 5,5 | | | | | | | |
| **Viskosität** Brookfield, RT, 10 upm, Spindel 4 | 4000 - 8000 mPas | | | | | | | |
| **Rest-Naßkämmbarkeit** | 89 | 80 | 91 | 96 | 92 | 82 | 77 | 108 |
| **Handwaschtest** | + | +++ | + | ++ | ++ | +++ | +++ | - |
| **Trübung** | klar | klar | klar | trüb | klar | trans. | trüb | klar |
| (-) kein Effekt; (+) leichte Rückfettung; (++) gute Rückfettung; (+++) starker Effekt | | | | | | | | |

| | | | | | | | | |
|---|---|---|---|---|---|---|---|---|
| * Vergleich | | | | | | | | |

Zur Untersuchung der Haaravivage und des rückfettenden Verhaltens wurden Haarsträhnen vor der Nullmessung mediumblondiert. Die Trockenkämmbarkeit wurde unter Zulassung der elektrostatischen Aufladung untersucht. Nach einer Einwirkzeit von 5 min wurden die Testlösungen (1g/1g Haar) unter Standardbedingungen (38 °C, 1l/min) 1 min gespült. Die Messung wurde an 20 Haarsträhnen durchgeführt. Eine ausführliche Beschreibung der Meßmethoden befindet sich in **J.Soc.Cosm.Chem., 24, 782 (1973).** Die Ergebnisse sind in Tabelle 4 zusammengefaßt. Angegeben ist jeweils die Restarbeit bzw. Restaufladung bezogen auf den Ausgangswert. Die Beurteilung des Haarglanzes erfolgte auf einer Skala von 1 bis 5. Die Beispiele 1 bis 4 sind erfindungsgemäß, die Versuche V1 und V2 dienen zum Vergleich.

**Tabelle 4:**

| **Nanoskalige Wachszubereitungen - Mengenangaben in Gew.-% -** | | | | | | |
|---|---|---|---|---|---|---|
| **Zusammensetzung/Performance** | **1** | **2** | **3** | **4** | **V1** | **V2** |
| **Sodium Laureth Sulfate** | 5,5 | 5,5 | 5,5 | 5,5 | 5,5 | 5,5 |
| **Ammonium Laureth Sulfate** | 2,4 | 2,4 | 2,4 | 2,4 | 2,4 | 2,4 |
| **Cocamide DEA** | 1,5 | 1,5 | 1,5 | 1,5 | 1,5 | 1,5 |
| **Dimethicone** | - | 3 | 3 | 3 | - | 2 |
| **Sonnenblumenwachs** | - | - | - | - | 0,3 | |
| **Lanolin** | - | - | - | - - | | 2,5 |
| **Laureth-2** | 2 | - | 2 | 2 | 2 | 2 |
| **Sodium Chloride** | 0,2 | 0,3 | 1,0 | 0,5 | 0,5 | 0,5 |
| **Erfindungsgemäßes Wachs 6** | 0,3 | - | 2,5 | - | - | - |
| **Erfindungsgemäßes Wachs 7** | - | 2,5 | - | 0,3 | - | - |
| **Generol® 122 N (nanoskalig)** **Erfindungsgemäßes Wachs 8** | - | - | 5 | 4 | - | - |
| **Wasser** | ad 100 | | | | | |
| ***Rest-Naßkämmarbeit [%]*** | 62 | 63 | 54 | 54 | 89 | 94 |
| ***Rest-Trockenkämmarbeit [%]*** | 65 | 65 | 67 | 60 | 106 | 77 |
| ***Rest -Aufladung [%]*** | 65 | 59 | 56 | 72 | 110 | 76 |
| ***Trübung*** | trüb | trüb | trüb | trüb | klar | klar |
| ***Brillianz*** | 3,2 | 2,5 | 4 | 4 | 1,5 | 2 |
| ***Stabilität*** | ++ | ++ | + | ++ | + | - |
| ***Dermatologische Verträglichkeit*** | ++ | ++ | + | ++ | + | + |

Die nachfolgende Tabelle 5 enthält eine Reihe von Formulierungsbeispielen mit Wachs-Nanopartikeln.

**Tabelle 5:**

| **Kosmetische Zubereitungen (Wasser, Konservierungsmittel ad 100 Gew.-%)** | | | | | | | | | | |
|---|---|---|---|---|---|---|---|---|---|---|
| **Zusammensetzung (INCI)** | **1** | **2** | **3** | **4** | **5** | **6** | **7** | **8** | **9** | **10** |
| **Texapon® NSO** Sodium Laureth Sulfate | - | - | - | - | - | - | 38,0 | 38,0 | 25,0 | - |
| **Texapon® SB 3** Disodium Laureth Sulfosuccinate | - | - | - | - | - | - | - | - | 10,0 | - |
| **Plantacare® 818** Coco Glucosides | - | - | - | - | - | - | 7,0 | 7,0 | 6,0 | - |
| **Plantacare® PS 10** Sodium Laureth Sulfate (and) Coco Glucosides | - | - | - | - | - | - | - | - | - | 16,0 |
| **Dehyton® PK 45** Cocamidopropyl Betaine | - | - | - | - | - | - | - | - | 10,0 | - |
| **Dehyquart® A** Cetrimonium Chloride | 2,0 | 2,0 | 2,0 | 2,0 | 4,0 | 4,0 | - | - | - | - |
| **Dehyquart L® 80** Dicocoylmethylethoxymonium Methosulfate (and) Propylenglycol | 1,2 | 1,2 | 1,2 | 1,2 | 0,6 | 0,6 | - | - | - | - |
| **Eumulgin® B2** Ceteareth-20 | 0,8 | 0,8 | - | 0,8 | - | 1,0 | - | - | - | - |
| **Eumulgin® VL 75** Lauryl Glucoside (and) Polyglyceryl-2 Polyhydroxystearate (and) Glycerin | - | - | 0,8 | - | 0.8 | - | - | - | - | - |
| **Lanette® O** Cetearyl Alcohol | 2,5 | 2,5 | 2,5 | 2,5 | 3,0 | 2,5 | - | - | - | - |
| **Cutina® GMS** Glyceryl Stearate | 0,5 | 0,5 | 0,5 | 0,5 | 0,5 | 1,0 | - | - | - | - |
| **Cetiol® HE** PEG-7 Glyceryl Cocoate | 1,0 | - | - | - | - | - | - | - | 1,0 | |
| **Cetiol® PGL** Hexyldecanol (and) Hexyldecyl Laurate | - | 1,0 | - | - | 1,0 | - | - | - | - | - |
| **Cetiol® V** Decyl Oleate | - | - | - | 1,0 | - | - | - | - | - | - |
| **Eutanol® G** Octyldodecanol | - | - | 1,0 | - | - | 1,0 | - | - | - | - |
| **Nutrilan® Keratin W** Hydrolyzed Keratin | - | - | - | 2,0 | - | - | - | - | - | - |
| **Lamesoft® LMG** Glyoeryl Laurate (and) Potassium Cocoyl Hydrolyzed Collagen | - | - | - | - | - | - | 3,0 | 2,0 | 4,0 | - |
| **Euperlan® PK 3000 AM** Glycol Distearate (and) Laureth-4 (and) Cocamidopropyl Betaine | - | - | - | - | - | - | - | 3,0 | 5,0 | 5,0 |
| **Generol® 122 N** Soja Sterol | - | - | - | - | 1,0 | 1,0 | - | - | - | - |
| **Erfindungsgemäßes Wachs 7 (gem. Tabelle 1)** | 1,0 | - | - | 1,0 | - | 1,5 | 3,0 | - | - | - |
| **Erfindungsgemäßes Wachs 3 (gem. Tabelle 1)** | - | - | 0,5 | - | 1,0 | - | - | - | 0,5 | 5,0 |
| **Erfindungsgemäßes Wachs 6 (gem. Tabelle 1)** | - | 1,0 | - | 1,0 | - | 1,5 | - | 3,0 | - | - |
| **Copherol® 12250** Tocopherol Acetate | - | - | 0,1 | 0,1 | - | - | - | - | - | - |
| **Arlypon® F** Laureth-2 | - | - | - | - | - | - | 3,0 | 3,0 | 1,0 | - |
| **Sodium Chloride** | - | - | - | - | - | - | - | 1,5 | - | 1,5 |
| (1-4) Haarspülung, (5-6) Haarkur, (7-8) Duschbad, (9) Duschgel, (10) Waschlotion | | | | | | | | | | |

**Tabelle 6:**

| **Kosmetische Zubereitungen (Wasser, Konservierungsmittel ad 100 Gew.-%)- Fortsetzung** | | | | | | |
|---|---|---|---|---|---|---|
| **Zusammensetzung (INCI)** | **11** | **12** | **13** | **14** | **15** | **20** |
| **Texapon® NSO** Sodium Laureth Sulfate | 20,0 | 20,0 | 12,4 | - | 25,0 | - |
| **Texapon® K 14 S** Sodium Myreth Sulfate | - | - | - | - | - | 23,0 |
| **Texapon® SB 3** Disodium Laureth Sulfosuocinate | - | - | - | - | - | - |
| **Plantacare® 818** Coco Glucosides | 5,0 | 5,0 | 4,0 | - | - | 4,0 |
| **Plantacare® 2000** Decyl Glucoside | - | - | - | - | 5,0 | - |
| **Plantacare® PS 10** Sodium Laureth Sulfate (and) Coco Glucosides | - | - | - | 40,0 | - | - |
| **Dehyton® PK 45** Cocamidopropyl Betaine | 20,0 | 20,0 | - | - | 8,0 | 7,0 |
| **Eumulgin® B1** Ceteareth-12 | - | - | - | - | 1,0 | - |
| **Eumulgin® B2** Ceteareth-20 | - | - | - | 1,0 | - | - |
| **Lameform® TGI** Polyglyceryl-3 Isostearate | - | - | - | 4,0 | - | - |
| **Dehymuls® PGPH** Polyglyceryl-2 Dipolyhydroxystearate | - | - | 1,0 | - | - | - |
| **Monomuls® 90-L 12** Glyceryl Laurate | - | - | - | - | - | 1,0 |
| **Cetlol® HE** PEG-7 Glyceryl Cocoate | - | 0,2 | - | - | - | - |
| **Eutanol® G** Octyldodecanot | - | - | - | 3,0 | - | - |
| **Nutrilan® Keratin W** Hydrolyzed Keratin | - | - | - | - | - | 2,0 |
| **Nutrilan® I** Hydrolyzed Collagen | 1,0 | - | - | - | - | - |
| **Lamesoft® LMG** Glyceryl Laurate (and) Potassium Cocoyl Hydrolyzed Collagen | - | - | - | - | - | - |
| **Lamesoft® 156** Hydrogenated Tallow Gyceride (and) Potassium Cocoyl Hydrolyzed Collagen | - | - | - | - | - | 5,0 |
| **Gluadin® WK** Sodium Cocoyl Hydrolyzed Wheat Protein | 1,0 | 1,5 | 4,0 | 1,0 | 3,0 | - |
| **Euperlan® PK 3000 AM** Glycol Distearate (and) Laureth-4 (and) Cocamidopropyl Betaine | 5,0 | 3,0 | 4,0 | - | - | - |
| **Panthenol** | - | - | 1,0 | - | - | - |
| **Arlypon® F** Laureth-2 | 2,6 | 1,6 | - | 1,0 | 1,5 | - |
| **Erfindungsgemäßes Wachs 1 (gem. Tabelle 1)** | 1,0 | 0,5 | - | - | - | 0,5 |
| **Erfindungsgemäßes Wachs 8 (gem. Tabelle 1)** | - | - | 0,2 | 0,4 | 1,0 | 0,1 |
| **Sodium Chloride** | - | - | - | - | - | 3,0 |
| **Glycerin (86 Gew.-%ig)** | - | 5,0 | - | - | - | - |
| (11-14) Duschbad Two-in-One), (15,20) Shampoo | | | | | | |

**Tabelle 7:**

| **Kosmetische Zubereitungen (Wasser, Konservierungsmittel ad 100 Gew.-%) - Fortsetzung** | | | |
|---|---|---|---|
| **Zusammensetzung (INCI)** | **21** | **22** | **23** |
| **Texapon® NSO** Sodium Laureth Sulfate | - | 30,0 | 30,0 |
| **Plantacare® 818** Coco Glucosides | - | 10,0 | - |
| **Plantacare® PS 10** Sodium Laureth Sulfate (and) Coco Glucosides | 22,0 | - | 5,0 |
| **Dehyton® PK 45** Cocamidopropyl Betaine | 15,0 | 10,0 | 15,0 |
| **Nutrilan® Elastin E20** Hydrolyzed Elastin | - | - | - |
| **Nutrilan® I-50** Hydrolyzed Collagen | - | - | - |
| **Gluadin® AGP** Hydrolyzed Wheat Gluten | 0,5 | 0,5 | 0,5 |
| **Gluadin® WK** Sodium Cocoyl Hydrolyzed Wheat Protein | 2,0 | 2,0 | 2,0 |
| **Euperlan® PK 3000 AM** Glycol Distearate (and) Laureth-4 (and) Cocamidopropyl Betaine | 5,0 | - | - |
| **Arlypon® F** Laureth-2 | - | - | - |
| **Erfindungsgemäßes Wachs 2 (gem. Tabelle 1)** | 1,0 | - | - |
| **Erfindungsgemäßes Wachs 4 (gem. Tabelle 1)** | - | 1,0 | 2,0 |
| **Glycerin (86 Gew.-%ig)** | - | - | - |
| (21-23) Schaumbad | | | |

## Patentansprüche

1. Verwendung von nanoskaligen Wachsen mit Teilchendurchmessem im Bereich von 10 bis 300 nm, welche von einem Schutzkolloid ummantelt vorliegen, das ausgewählt ist aus der Gruppe Gelatine, Casein, Gummi arabicum, Lysalbinsäure, Stärke, Polyvinylalkohole, Polyvinylpyrrolidone, Polyalkylenglycole und Polyacrylate, zur Herstellung von oberflächenaktiven Zubereitungen.

2. Verwendung nach Anspruch 1, **dadurch gekennzeichnet, daß** man synthetische und/oder natürliche Wachse und/oder Wachsester der Formel **(I),**
**R**^{**1**}**COO-R**^{**2**} (I)
in der R'CO für einen linearen oder verzweigten Acylrest mit 6 bis 22 Kohlenstoffatomen mit 0 und/oder 1 bis 3 Doppelbindungen und R² für einen linearen oder verzweigten Alkyl- und/oder Alkenylrest mit 6 bis 22 Kohlenstoffatomen steht, mit der Maßgabe, daß die Anzahl der Kohlenstoffatome im Ester mindestens 20 beträgt, einsetzt.

3. Verwendung nach den Ansprüchen 1 und/oder 2, **dadurch gekennzeichnet, daß** man Siliconwachse einsetzt, die ausgewählt sind aus der Gruppe, die gebildet wird von Dimethylpolysiloxanen, Methylphenyl-polysiloxanen, cyclischen Siliconen sowie amino-, fettsäure-, alkohot-, polyether-, epoxy-, fluor-, glykosid- und/oder alkylmodifizierten Siliconverbindungen.

4. Verwendung nach mindestens einem der Ansprüche 1 bis 3, **dadurch gekennzeichnet, daß** man Perlglanzwachse einsetzt, die ausgewählt sind aus der Gruppe, die gebildet wird von Alkylenglycolester, Fettsäurealkanolamide, Partialglyceride, Ester von mehrwertigen, gegebenenfalls hydroxysubstituierte Carbonsäuren mit Fettalkoholen mit 6 bis 22 Kohlenstoffatomen, Fettstoffe, Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

5. Verwendung nach mindestens einem der Ansprüche 1 bis 4, **dadurch gekennzeichnet, daß** man nanoskalige Wachse einsetzt, die man erhält, indem man
(a) die Ausgangsstoffe unter überkritischen oder nahekritischen Bedingungen in einem geeigneten Lösungsmittel löst,
(b) die fluide Mischung über eine Düse in ein Vakuum, ein Gas oder eine Flüssigkeit entspannt, und
(c) das Lösemittel dabei gleichzeitig verdampft.

6. Verwendung nach mindestens einem der Ansprüche 1 bis 5, **dadurch gekennzeichnet, daß** man die Wachse in Mengen von 0,1 bis 5 Gew.-% - bezogen auf die Zubereitungen - einsetzt.

7. Verwendung nach mindestens einem der Ansprüche 1 bis 6, **dadurch gekennzeichnet, daß** man die Wachse als Rückfettungsmittel zur Herstellung von oberflächenaktiven Zubereitungen einsetzt.

8. Verwendung nach mindestens einem der Ansprüche 1 bis 7, **dadurch gekennzeichnet, daß** man die Wachse als Avivagemittel zur Herstellung von oberflächenaktiven Zubereitungen als Haarbehandlungsmitteln einsetzt.

9. Verwendung nach mindestens einem der Ansprüche 1 bis 8, **dadurch gekennzeichnet, daß** man die Wachse als Trübungsmittel zur Herstellung von oberflächenaktiven Zubereitungen einsetzt.

## Claims

1. The use of nanoscale waxes with particle diameters of 10 to 300 nm coated with a protective colloid selected from the group consisting of gelatine, casein, gum arabic, lysalbinic acid, starch, polyvinyl alcohols, polyvinyl pyrrolidones, polyalkylene glycols and polyacrylates for the production of surface-active preparations.

2. The use claimed in claim 1, **characterized in that** synthetic and/or natural waxes and/or wax esters corresponding to formula (I):
**R**^{**1**}**COO-R**^{**2**} (I)
in which R¹CO is a linear or branched acyl group containing 6 to 22 carbon atoms and 0 and/or 1 to 3 double bonds and R² is a linear or branched alkyl and/or alkenyl group containing 6 to 22 carbon atoms, with the proviso that the number of carbon atoms in the ester is at least 20,
are used.

3. The use claimed in claims 1 and/or 2, **characterized in that** silicone waxes selected from the group consisting of dimethyl polysiloxanes, methylphenyl polysiloxanes, cyclic silicones and amino-, fatty-acid-, alcohol-, polyether-, epoxy-, fluorine-, glycoside- and/or alkyl-modified silicone compounds are used.

4. The use claimed in at least one of claims 1 to 3, **characterized in that** pearlizing waxes selected from the group consisting of alkylene glycol esters, fatty acid alkanolamides, partial glycerides, esters of polybasic, optionally hydroxysubstituted carboxylic acids with C₆₋₂₂ fatty alcohols, fatty compounds, fatty acids, such as stearic acid, hydroxystearic acid or behenic acid, ring opening products of C₁₂₋₂₂ olefin epoxides with C₁₂₋₂₂ fatty alcohols and/or C₂₋₁₅ polyols containing 2 to 10 hydroxyl groups and mixtures thereof are used.

5. The use claimed in at least one of claims 1 to 4, **characterized in that** nanoscale waxes obtained by
(a) dissolving the starting materials in a suitable solvent under supercritical or near-critical conditions,
(b) expanding the fluid mixture through a nozzle into a vacuum, a gas or a liquid and
(c) simultaneously evaporating the solvent
are used.

6. The use claimed in at least one of claims 1 to 5, **characterized in that** the waxes are used in quantities of 0.1 to 5% by weight, based on the preparations.

7. The use claimed in at least one of claims 1 to 6, **characterized in that** the waxes are used as lipid layer enhancers for the production of surface-active preparations.

8. The use claimed in at least one of claims 1 to 7, **characterized in that** the waxes are used as conditioners in the production of surface-active preparations in the form of hair treatment compositions.

9. The use claimed in at least one of claims 1 to 8, **characterized in that** the waxes as used as opacifiers in the production of surface-active preparations.

## Revendications

1. Utilisation de nanocires dont le diamètre particulaire est compris entre 10 et 300 nm, enrobées d'un colloïde protecteur choisi dans le groupe constitué des gélatines, de la caséine, de la gomme arabique, de l'acide lysalbique, des amidons, des alcools polyvinyliques, des polyvinylpyrrolidones, des polyalkylèneglycols et des polyacrylates, pour l'obtention de préparations tensioactives.

2. Utilisation selon la revendication 1,
**caractérisée en ce qu'**
on met en oeuvre des cires synthétiques et/ou naturelles et/ou des esters de cires correspondant à la formule (I)
R¹COO-R² (I)
dans laquelle R¹CO représente un radical acyle linéaire ou ramifié comportant de 6 à 22 atomes de carbone et 0 et/ou de 1 à 3 doubles liaisons et R² représente un radical alkyle et/ou alcényle linéaire ou ramifié comportant de 6 à 22 atomes de carbone étant précisé que le nombre d'atomes de carbone de l'ester est d'au moins 20.

3. Utilisation selon les revendications 1 et/ou 2,
**caractérisée en ce qu'**
on met en oeuvre des cires de silicone choisies dans le groupe constitué des diméthylpolysiloxanes, méthylphénylpolysiloxanes, silicones cycliques ainsi que des composés de silicone modifiés par des amines acides gras, alcools, polyéthers, époxy, fluor, glycosides et/ou alkyles.

4. Utilisation selon au moins l'une des revendications 1 à 3,
**caractérisée en ce qu'**
on met en oeuvre des cires nacrées choisies dans le groupe constitué des esters d'alkylèneglycol, des alcanolamides d'acides gras, des glycérides partiels, des esters d'acides carboxyliques polyvalents, éventuellement hydroxy substitués et d'alcools gras comportant de 6 à 22 atomes de carbone, des matières grasses, des acides gras tels que l'acide stéarique, l'acide hydroxystéarique ou l'acide béhénique, des produits d'ouverture de cycle d'époxydes oléfiniques comportant de 12 à 22 atomes de carbone avec des alcools gras comportant de 12 à 22 atomes de carbone et/ou des polyols comportant de 2 à 15 atomes de carbone et de 2 à 10 groupes hydroxyle ainsi que leurs mélanges.

5. Utilisation selon au moins l'une des revendications 1 à 4,
**caractérisée en ce qu'**
on met en oeuvre des nanocires obtenues par les étapes suivantes :
(a) dissolution des produits de départ dans un solvant approprié dans des conditions surcritiques ou quasi-critiques,
(b) détente du mélange fluide par l'intermédiaire d'une buse dans le vide, un gaz ou un liquide, et
(c) vaporisation simultanée du solvant.

6. Utilisation selon au moins l'une des revendications 1 à 5,
**caractérisée en ce qu'**
on met en oeuvre les cires dans des proportions allant de 0,1 à 5 % en poids par rapport aux préparations.

7. Utilisation selon au moins l'une des revendications 1 à 6,
**caractérisée en ce qu'**
on met en oeuvre les cires en tant qu'agents surgras pour l'obtention de préparations tensioactives.

8. Utilisation selon au moins l'une des revendications 1 à 7,
**caractérisée en ce qu'**
on met en oeuvre les cires en tant qu'agents d'adoucissage pour l'obtention de préparations tensioactives correspondant à des agents de traitement capillaire.

9. Utilisation selon au moins l'une des revendications 1 à 8,
**caractérisée en ce qu'**
on met en oeuvre les cires en tant qu'agents opacifiants pour l'obtention de préparations tensioactives.
